# EUROPÄISCHE PATENTSCHRIFT

(11) **EP 1 226 143 B1**
(45) Veröffentlichungstag und Bekanntmachung des Hinweises auf die Patenterteilung: **02.04.2003**
(21) Anmeldenummer: 00975916.8
(22) Anmeldetag: 25.10.2000
(51) Int. Cl.: C07D 471/04, A61K 31/437, A61P 11/00

(54) **IMIDAZOPYRIDINDERIVATE ALS PHOSPHODIESTERASE VII-HEMMER**
IMIDAZOPYRIDINE DERIVATIVES USED AS PHOSPHODIESTERASE VII INHIBITORS
DERIVES D'IMIDAZOPYRIDINE COMME INHIBITEURS DE PHOSPHODIESTERASE VII

(30) Priorität: 06.11.1999 DE 19953414
(43) Veröffentlichungstag der Anmeldung: 31.07.2002
(73) Patentinhaber: MERCK PATENT GmbH, 64293 Darmstadt (DE)
(72) Erfinder: EGGENWEILER, Hans, Michael, 64331 Weiterstadt (DE); ACKERMANN, Karl-August, 64372 Ober-Ramstadt (DE); JONAS, Rochus, 64291 Darmstadt (DE); WOLF, Michael, 64297 Darmstadt (DE); GASSEN, Michael, 64347 Griesheim (DE); WELGE, Thomas, 64665 Alsbach (DE)
(86) Internationale Anmeldenummer: EP0010525
(87) Internationale Veröffentlichungsnummer: WO01034601

(56) Entgegenhaltungen:
- EP-A- 0 426 467
- SEMONSKY, M. ET AL: "Substanzen mit antineoplasischer Wirkung" ARZNEIMITTEL FORSCHUNG. DRUG RESEARCH., Bd. 20, Nr. 3, 1970, Seiten 316-23, XP002170611 EDITIO CANTOR. AULENDORF., DE ISSN: 0004-4172

## Beschreibung

Die Erfindung betrifft Verbindungen der Formel I 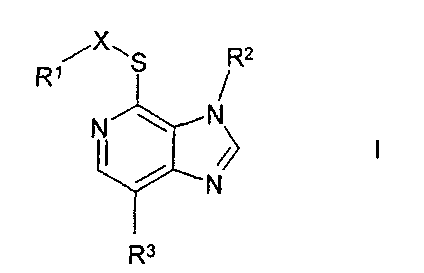 worin
- R¹: CONR⁴R⁵,
- R²: H oder A,
- R⁴, R⁵: jeweils unabhängig voneinander H oder A¹,
- R³: Hal,
- Hal: F, Cl, Br oder I,
- A: Alkyl mit 1-4 C-Atomen,
- A¹: Alkyl mit 1-10 C-Atomen,
- X: Alkylen mit 1-4 C-Atomen, wobei eine Ethylengruppe auch durch eine Doppel- oder Dreifachbindung ersetzt sein kann,
bedeuten,
sowie deren physiologisch unbedenklichen Salze und/oder Solvate.

Andere Imidazopyridinderivate mit GABA-agonistischen Wirkungen sind beispielsweise aus der EP 82369 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.
Insbesondere zeigen sie eine spezifische Inhibierung der "Rolipram insensitiven" cAMP Phosphodiesterase (PDE VII).

Die biologische Aktivität der Verbindungen der Formel I kann nach Methoden bestimmt werden, wie sie z.B von M.A. Giembycz et al. in Br. J. Pharmacol. (1996), **118,** 1945-1958 beschrieben sind.
Die Affinität der Verbindungen für cAMP-Phosphodiesterase (PDE VII) wird durch die Ermittlung ihrer IC₅₀-Werte (Konzentration des Inhibitors, die benötigt wird, um eine 50 %ige Inhibierung der Enzymaktivität zu erreichen) bestimmt.
Zur Durchführung der Bestimmungen wurden anstelle von T-Lymphozyten homogenisierte SK-N-SH Neuroblastomazellen verwendet, zur PDE III Inhibierung wurde Cl-930 eingesetzt. Hierbei handelt es sich um einen selektiven PDE III Inhibitor (J.A. Bristol et al., J. Med. Chem. 1984, 27(9), 1099-1101).
Alternativ wird SK-N-SH durch HUT-78 ersetzt und statt Cl-930 wird mit Trequensin inhibiert (D. Ruppert et al., Life Sci. 31:2037, 1982).

Die Verbindungen der Formel I können zur Behandlung von asthmatischen Erkrankungen eingesetzt werden.
Die antiasthmatische Wirkung kann z. B. analog der Methode von T. Olsson, Acta allergologica **26,** 438-447 (1971), bestimmt werden.

Da cAMP knochenabbauende Zellen hemmt und knochenaufbauende Zellen stimuliert (S. Kasugai et al., M 681 und K. Miyamoto, M 682, in Abstracts of the American Society for Bone and Mineral Research 18^{th} Annual Meeting, 1996), können die Verbindungen der Formel I zur Behandlung von Osteoporose eingesetzt werden.

Die Verbindungen zeigen außerdem eine antagonistische Wirkung auf die Produktion von TNFα (Tumor Nekrose Faktor) und eignen sich daher zur Behandlung von allergischen und entzündlichen Krankheiten, Autoimmunkrankheiten, wie z.B. rheumatoide Arthritis, multiple Sklerose, Morbus Crohn, Diabetes mellitus oder ulzerative Kolitis, Transplantatabstoßungsreaktionen, Kachexie und Sepsis.
Die antiinflammatorische Wirkung der Substanzen der Formel I und ihre Wirksamkeit zur Behandlung von z.B. Autoimmunerkrankungen wie multipler Sklerose oder rheumatoider Arthritis, kann analog den Methoden von N. Sommer et al., Nature Medicine **1,** 244-248 (1995) oder L. Sekut et al., Clin. Exp. Immunol. **100**, 126-132 (1995) bestimmt werden.

Die Verbindungen können zur Behandlung von Kachexie eingesetzt werden. Die anti-kachektische Wirkung kann in TNF-abhängigen Modellen der Kachexie geprüft werden (P. Costelli et al., J. Clin. Invest. **95,** 2367ff. (1995); J.M. Argiles et al., Med. Res. Rev. **17,** 477ff. (1997)).

Die PDE VII-Inhibitoren können auch das Wachstum von Tumorzellen hemmen und sind deshalb für die Tumortherapie geeignet (für PDE IV-Hemmer vgl. D. Marko et al., Cell Biochem. Biophys. **28,** 75ff. (1998)).

Sie können weiterhin für die Therapie von Sepsis und zur Behandlung von Gedächtnisstörungen, Atherosklerose, atopische Dermatitis und AIDS eingesetzt werden, ferner zur Behandlung T-Zellen-abhängiger Krankheiten (L.Li et al., Science, 1999, 283, 848-851).

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden. Insbesondere können die Verbindungen der Formel I als Arzneimittelwirkstoffe zur PDE VII Inhibierung in der Human- und Veterinärmedizin eingesetzt werden.

Die Erfindung betrifft weiterhin die Verwendung der Verbindungen der Formel I zur Herstellung eines Arzneimittels zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

A bedeutet Alkyl mit 1-4 C-Atomen und hat 1, 2, 3 oder 4 C-Atome und bedeutet vorzugsweise Methyl, Ethyl oder Propyl, weiterhin bevorzugt Isopropyl, Butyl, Isobutyl, sek.-Butyl oder tert.-Butyl. In den Resten können auch 1-7 H-Atome durch F und/oder Cl ersetzt sein. A bedeutet daher auch z.B. Trifluormethyl oder Pentafluorethyl.

A¹ bedeutet Alkyl mit 1-10 C-Atomen und hat 1, 2, 3, 4, 5, 6, 7, 8, 9 oder 10 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, Neopentyl, Isopentyl oder Hexyl. In den Resten können auch 1-7 H-Atome durch F und/oder Cl ersetzt sein. A¹ bedeutet daher auch z.B. Trifluormethyl oder Pentafluorethyl.

X bedeutet Alkylen mit 1-4 C-Atomen, vorzugsweise Methylen, Ethylen, Propylen oder Butylen, wobei eine Ethylengruppe auch durch eine Doppeloder Dreifachbindung ersetzt sein kann. X bedeutet daher auch z.B. -CH₂-CH=CH-CH₂- oder -C≡C-.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden-Teilformeln la bis Ic ausgedrückt werden, die der Formel I entsprechen und worin die nicht näher bezeichneten Reste die bei der Formel I angegebene Bedeutung haben, worin jedoch
- in la R³: Cl bedeutet;
- in Ib R³: Cl,
- X: Alkylen mit 1-4 C-Atomen bedeutet;
- in Ic R³: Cl,
- X: Alkylen mit 1, 2, 3 oder 4 C-Atomen,
- A¹: Alkyl mit 1, 2, 3 oder 4 C-Atomen bedeutet.

Die Verbindungen der Formel I und auch die Ausgangsstoffe zu ihrer Herstellung werden insbesondere analog hergestellt wie in der EP 82369 auf Seite 3, linke Spalte, Zeile 18 bis Seite 4, Spalte 6, Zeile 16 oder in Beispiel 1 beschrieben.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung sind auch Arzneimittel der Formel I und ihre physiologisch unbedenklichen Salze als Phosphodiesterase VII-Hemmer.

Gegenstand der Erfindung sind ferner pharmazeutische Zubereitungen, enthaltend mindestens einen Phosphodiesterase VII-Hemmer der Formel I und/oder eines ihrer physiologisch unbedenklichen Salze und/oder Solvate zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

Dabei werden die Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die pharmazeutischen Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Gegenstand der Erfindung sind insbesondere die in den nachstehenden Beispielen aufgeführten Verbindungen der Formel I sowie deren physiologisch unbedenklichen Salze und/oder Solvate als PDE VII-Hemmer sowie deren Verwendung zur Herstellung eines Arzneimittels zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

### Beispiele:

2-(3-Butyl-7-chlor-3*H*-imidazo[4,5-*c*]pyridin-4-ylsulfanyl)-*N*,*N*-dimethylacetamid 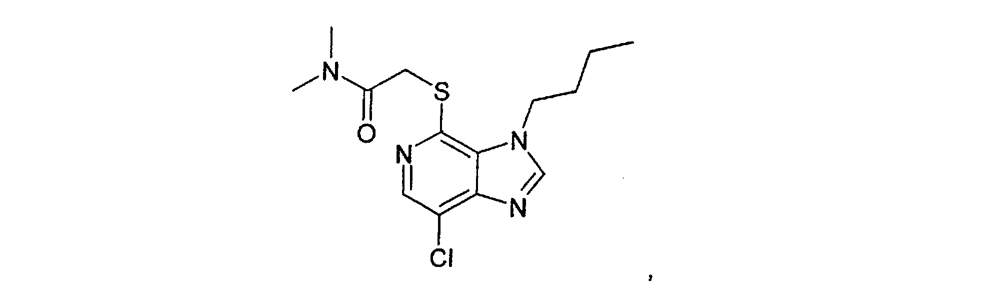
2-(3-Butyl-7-chlor-3*H*-imidazo[4,5-*c*]pyridin-4-ylsulfanyl)-acetamid,
2-(3-Butyl-7-chlor-3*H*-imidazo[4,5-*c*]pyridin-4-ylsulfanyl)-propionamid,
2-(3-Butyl-7-chlor-3*H*-imidazo[4,5-*c*]pyridin-4-ylsulfanyl)-butyramid,
2-(3-Butyl-7-chlor-3*H*-imidazo[4,5-*c*]pyridin-4-ylsulfanyl)-*N*-hexyl-acetamid,
2-(3-Propyl-7-chlor-3*H*-imidazo[4,5-*c*]pyridin-4-ylsulfanyl)-*N*-octyl-acetamid,
4-(3-Butyl-7-chlor-3*H*-imidazo[4,5-*c*]pyridin-4-ylsulfanyl)-but-2-ensäuredimethylamid.

Die nachfolgenden Beispiele betreffen pharmazeutische Zubereitungen:

### Beispiel A: Injektionsgläser

Eine Lösung von 100 g eines Phosphodiesterase VII-Hemmers der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Phosphodiesterase VII-Hemmers der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Phosphodiesterase VII-Hemmers der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Phosphodiesterase VII-Hemmers der Formel I mit 99,5 g Vaseline unter aseptischen Bedingungen.

### Beispiel E: Tabletten

Ein Gemisch von **1** kg Phosphodiesterase VII-Hemmer der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Phosphodiesterase VII-Hemmer der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Phosphodiesterase VII-Hemmer der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Phosphodiesterase VII-Hemmer der Formel I in 10 I isotonischer NaCI-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 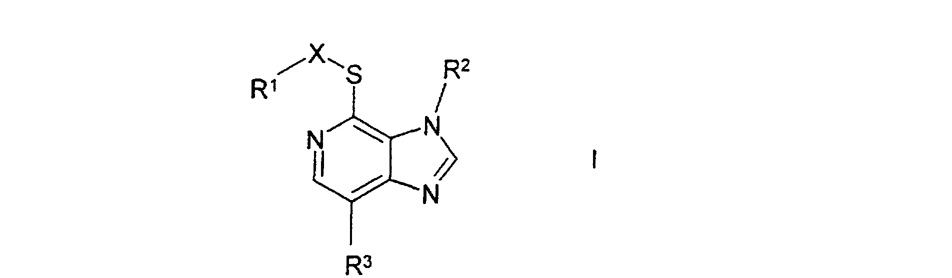 worin
R¹ CONR⁴R⁵,
R² H oder A,
R⁴, R⁵ jeweils unabhängig voneinander H oder A¹,
R³ Hal,
Hal F, Cl, Br oder I,
A Alkyl mit 1-4 C-Atomen,
A¹ Alkyl mit 1-10 C-Atomen,
X Alkylen mit 1-4 C-Atomen, wobei eine Ethylengruppe auch durch eine Doppel- oder Dreifachbindung ersetzt sein kann,
bedeuten,
sowie deren physiologisch unbedenklichen Salze und/oder Solvate.

2. Imidazopyridinderivate der Formel I gemäß Anspruch 1 sowie deren physiologisch unbedenklichen Salze und Solvate als Arzneimittel.

3. Arzneimittel nach Anspruch 2 zur Inhibierung der Phosphodiesterase VII.

4. Arzneimittel nach Anspruch 3 zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

5. Pharmazeutische Zubereitung, enthaltend mindestens ein Arzneimittel gemäß einem der Ansprüche 3 oder 4 sowie gegebenenfalls Träger- und/oder Hilfsstoffe und gegebenenfalls andere Wirkstoffe.

6. Verwendung von Verbindungen der Formel I gemäß Anspruch 1 und/oder ihre physiologisch unbedenklichen Salze oder Solvate zur Herstellung eines Arzneimittels zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

## Claims

1. Compounds of the formula I 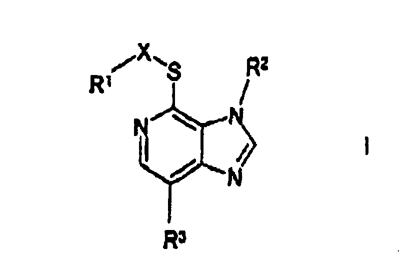 in which
R¹ is CONR⁴R⁵,
R² is H or A,
R⁴ and R⁵ are each, independently of one another, H or A¹,
R³ is Hal,
Hal is F, Cl, Br or I,
A is alkyl having 1-4 carbon atoms,
A¹ is alkyl having 1-10 carbon atoms,
X is alkylene having 1-4 carbon atoms, in which one ethylene group may also be replaced by a double or triple bond,
and physiologically acceptable salts and/or solvates thereof.

2. Imidazopyridine derivatives of the formula I according to Claim 1 and physiologically acceptable salts and solvates thereof as medicaments.

3. Medicament according to Claim 2 for inhibiting phosphodiesterase VII.

4. Medicament according to Claim 3 for combating allergic diseases, asthma, chronic bronchitis, atopic dermatitis, psoriasis and other skin diseases, inflammatory diseases, autoimmune diseases, such as, for example, rheumatoid arthritis, multiple sclerosis, Crohn's disease, diabetes mellitus or ulcerative colitis, osteoporosis, transplant rejecttion reactions, cachexia, tumour growth or tumour metastases, sepsis, memory disorders, atherosclerosis and AIDS.

5. Pharmaceutical preparation comprising at least one medicament according to one of Claims 3 or 4 and, if desired, excipients and/or adjuvants and, if desired, other active ingredients.

6. Use of compounds of the formula I according to Claim 1 and/or physiologically acceptable salts or solvates thereof for the preparation of a medicament for combating allergic diseases, asthma, chronic bronchitis, atopic dermatitis, psoriasis and other skin diseases, inflammatory diseases, autoimmune diseases, such as, for example, rheumatoid arthritis, multiple sclerosis, Crohn's disease, diabetes mellitus or ulcerative colitis, osteoporosis, transplant rejection reactions, cachexia, tumour growth or tumour metastases, sepsis, memory disorders, atherosclerosis and AIDS.

## Revendications

1. Composés de formule I 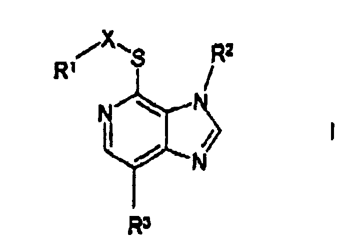 dans laquelle
R¹ représente CONR⁴R⁵,
R² représente H ou A,
R⁴ et R⁵ représentent chacun, indépendamment l'un de l'autre, H ou A¹,
R³ représente Hal,
Hal représente F, Cl, Br ou I,
A représente alkyle ayant de 1 à 4 atomes de carbone,
A¹ représente alkyle ayant de 1 à 10 atomes de carbone,
X représente alkylène ayant de 1 à 4 atomes de carbone, dans lequel un groupement éthylène peut également être remplacé par une liaison double ou triple,
et les sels et/ou solvats physiologiquement acceptables de ceux-ci.

2. Dérivés d'imidazopyridine de formule I selon la revendication 1 et les sels et solvats physiologiquement acceptables de ceux-ci comme médicaments.

3. Médicament selon la revendication 2, pour l'inhibition de la phosphodiestérase VII.

4. Médicament selon la revendication 3, pour lutter contre les maladies allergiques, l'asthme, la bronchite chronique, la dermatite atopique, le psoriasis et d'autres maladies de la peau, les maladies inflammatoires, les maladies auto-immunes, telles que, par exemple, la polyarthrite rhumatoïde, la sclérose en plaques, la maladie de Crohn, le diabète sucré ou la rectocolite hémorragique, l'ostéoporose, les réactions de rejet de greffon, la cachexie, la croissance de tumeurs ou les métastases tumorales, la septicémie, les troubles de la mémoire, l'athérosclérose et le SIDA.

5. Préparation pharmaceutique, comprenant au moins un médicament selon l'une des revendications 3 ou 4 et, si on le désire, des excipients et/ou des adjuvants et, si on le désire, d'autres ingrédients actifs.

6. Utilisation de composés de formule I selon la revendication 1 et/ou de sels ou solvats physiologiquement acceptables de ceux-ci pour la préparation d'un médicament pour lutter contre les maladies allergiques, l'asthme, la bronchite chronique, la dermatite atopique, le psoriasis et d'autres maladies de la peau, les maladies inflammatoires, les maladies auto-immunes, telles que, par exemple, la polyarthrite rhumatoïde, la sclérose en plaques, la maladie de Crohn, le diabète sucré ou la rectocolite hémorragique, l'ostéoporose, les réactions de rejet de greffon, la cachexie, la croissance de tumeurs ou les métastases tumorales, la septicémie, les troubles de la mémoire, l'athérosclérose et le SIDA.
